# EUROPEAN PATENT APPLICATION

(11) **EP 0 761 242 A1**
(43) Date of publication of application: **12.03.1997**
(21) Application number: 96202314.9
(22) Date of filing: 19.08.1996
(51) Int. Cl.: A61L 27/00, A61F 2/36, A61F 2/34

(54) **Orthopaedic implant with bearing surface**

(30) Priority: 21.08.1995 US 517511
(71) Applicant: Bristol-Myers Squibb Company, New York, N.Y. 10154 (US)
(72) Inventor: Rohr, William L., Jr., Warsaw, IN 46580 (US); Swarts, Dale F., Warsaw, IN 46580 (US); Devanathan, Thirumalai, Warsaw, IN 46580 (US); Krebs, Steven L., Fort Wayne, IN 46804 (US); Nazre, Aniruddha A., 4500 Solothurn (CH)
(74) Representative: Mays, Julie

(57) **Abstract**

This invention includes an orthopaedic implant, which defines a bearing surface for articulating contact with a second implant or the patient's bone. The implant is formed from a molded semi crystallin thermal plastic polymer, preferably a poly aryl ether ketone. The poly aryl ether ketone material is preferably injection molded and forms the bearing surface for the implant. The implant may take the shape of any number of orthopaedic bearing surfaces such as an acetabular cup for articulation against either a prosthetic or natural femoral head, or as a bearing surface for the tibial for articulation against the distal femur or femoral component of a total knee prosthesis system. A porous metal layer may be attached to the implant.

## Description

### Field of the Invention

This invention relates to an orthopaedic implant and has special relevance to an orthopaedic implant with a bearing surface formed from a thermoplastic polymer.

### Background of the Invention

It is well known in the field of orthopaedic implants to use ultra high molecular weight poly ethylene (UHMWPE) as the material of choice for an acetabular cup liner or a tibial bearing plate. Typically, these components are held in a fixed relationship relative to the bone and accommodate a second component. The second component, such as a prosthetic femoral head in the case of an acetabular cup, or a femoral knee component in the case of a tibial bearing plate, articulates or otherwise slides against the UHMWPE component at the UHMWPE bearing surface. In laboratory experiments, it has been shown that during articulation a portion of the UHMWPE may wear off the component and create wear debris. It is though that such wear debris may have a detrimental effect on the long term success of the implant.

It has recently become known in the field of orthopaedics to use a semi-crystallin thermal plastic polymer, preferably a poly aryl ether ketone, as an adhesive to bond two implant components together. The poly aryl ether ketone is typically situated between a solid component and a porous surface layer to adhesively and mechanically bond the component parts together.

### Summary of the Invention

This invention includes an orthopaedic implant, which defines a bearing surface for articulating contact with a second implant or the patient's bone. The implant is formed from a semi crystallin thermal plastic polymer, preferably a poly aryl ether ketone sold by BASF Corporation under the trade name ULTRAPEK. The poly aryl ether ketone material is preferably injection molded and forms the bearing surface for the implant. The implant may take the shape of any number of orthopaedic bearing surfaces such as an acetabular cup for articulation against either a prosthetic or natural femoral head, or as a bearing surface for the tibial for articulation against the distal femur or femoral component of a total knee prosthesis system. It is thought that in laboratory testing, a bearing component formed from the ULTRAPEK poly aryl ether ketone material will generate less wear debris as compared to a similar component formed from UHMWPE. It is believed that this beneficial wear characteristic of the poly aryl ether ketone ULTRAPEK could lengthen the life of the orthopaedic implant in vivo.

In the case of an acetabular cup, the implant will be positioned in apposition with the prepared acetabulum of the patient. Therefore, mechanical fixation is required to affix the implant to the bone. In the preferred embodiment, the outer diameter of the molded implant will be plasma spray coated with a metal, such as titanium. The titanium will partially melt into the polymer implant and form an integral outer porous layer for contact with the bone.

In an alternative embodiment, the porous layer could be plasma sprayed onto a carbon tooling and then cooled. The porous shell could be inserted into an injection mold machine and molded to the thermal plastic polymer during the molding cycle.

The use of poly aryl ether ketone as a bearing surface for an implant is believed to improve the wear properties for the implant over the prior art implants and therefore increase the longevity of the implant.

Accordingly, it is an object of the invention to provide for an implant having a bearing surface formed from a semi crystallin thermal plastic polymer.

Another object of the invention is to provide for an implant having a bearing surface formed from poly aryl ether ketone.

Yet another object of the invention is to provide for an acetabular cup formed from a poly aryl ether ketone and having an outer diameter formed from a plasma sprayed metal coating.

Still other objects of the invention will become apparent upon a reading of the following description taken with the accompanying drawings.

### Brief Description of the Drawings

Fig. 1 is a perspective view of an implant of the invention illustrated as an acetabular cup component.

Fig. 2. is a cross section of the implant of Fig. 1.

Fig. 3 is a perspective view of an implant of the invention illustrated as a prosthetic tibial bearing surface.

### Description of the Preferred Embodiment

The preferred embodiments herein described are not intended to be exhaustive or to limit the application to the precise forms disclosed. Rather, they are chosen and described to enable others skilled in the art to utilize their teachings.

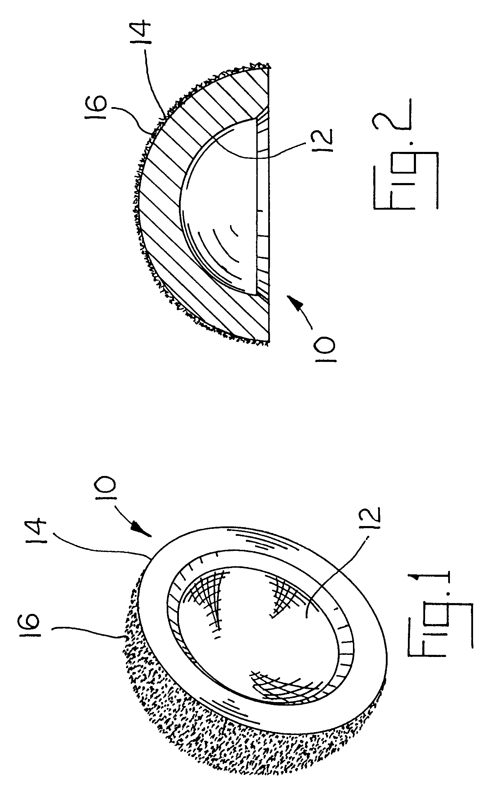

Referring now to the figures, a prosthetic acetabular cup 10 is illustrated in Figs. 1 and 2 and includes an inner substantially hemispherical cavity 12 (hereinafter referred to as the inner diameter or ID) configured for accommodating a prosthetic femoral head or the femoral head of the patient's femur (not shown). The cup also includes a substantially hemispherical outer periphery 14 hereinafter referred to as the outer diameter or OD. The cup is formed from a semi crystallin thermoplastic polymer material and is preferably a poly aryl ether ketone. The preferred poly aryl ether ketone is obtained from BASF Corporation and is sold under the trademark ULTRAPEK A3000 (hereinafter referred to as "the material"). The material has a melt temperature of around 700 degrees Fahrenheit (415 degrees Celsius). Preferably, the cup is formed by an injection molding process to form a very smooth inner diameter for accepting the articulating motion of the femoral head (not shown). As illustrated in the figures, the OD is covered by a porous metal surface 16. In the embodiment illustrated, the porous metal surface is applied by plasma flame spraying titanium, using a known process, onto the outer diameter after the injection molding process. Plasma spraying titanium requires process temperatures in excess of the melt temperature of the material. Therefore, during the process, the small particles of the titanium melt into the material a small amount and form a melt bond therewith to be securely fastened to the material. Alternatively, the titanium could be plasma sprayed onto a carbon tooling (not shown), cooled, and then placed within the cavity of an injection mold machine in order to form the cup illustrated in Figs. 1 and 2. As an alternative to plasma flame spraying, the porous metal layer could consist of a fiber metal pad preformed to shape and then inserted into the mold or a layer of small metal beads sintered into the appropriate shape and inserted into a mold.

Further, the porous surface layer could be omitted in its entirety and the OD of the cup could be configured for accommodation within a metal shell or for cement fixation into the prepared acetabulum. In the cup alternative, the cup made from the material would have a smooth ID and an OD adapted with notches or grooves for engagement by the metal shell or for direct cemented application to the prepared acetabulum.

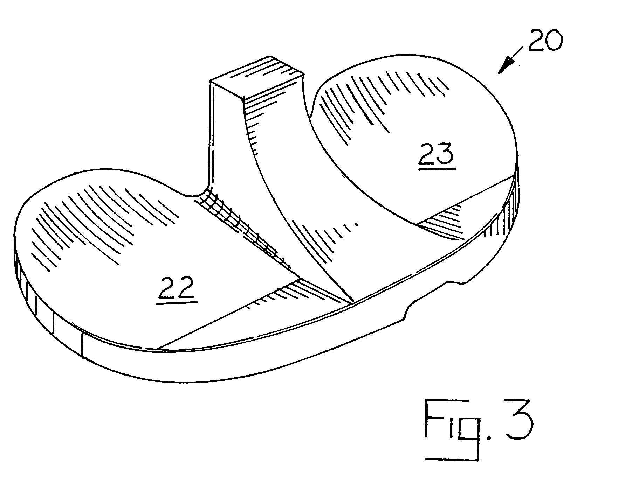

A tibial bearing plate 20 for connection to a prosthetic tibial plate is illustrated in Fig. 3 and includes articulating surfaces 22, 23 configured for contact with the condyles of an femoral knee joint component (not shown). The tibial plate would be fixed relative to the tibia and allow the femoral component to articulate or otherwise slide over the articulating surfaces 22, 23. Again, the tibial bearing plate is formed from a semi crystallin thermoplastic polymer, preferably a poly aryl ether ketone sold by BASF Corporation under the trademark ULTRAPEK..

It should be understood that the invention is not to be limited to the precise form disclosed but may be modified within the keeping of the appended claims.

## Claims

1. A molded orthopaedic implant defining a surface to accommodate articulation against a second component, wherein at least said surface is formed from a semi crystallin thermal plastic polymer.

2. The implant of Claim 1 wherein said semi crystallin thermal plastic polymer is a poly aryl ether ketone.

3. The implant of Claim 1 wherein the implant is formed from a semi crystallin thermal plastic polymer.

4. The implant of Claim 3 wherein said semi crystallin thermal plastic polymer is a poly aryl ether ketone.

5. The orthopaedic implant of Claim 1 wherein the implant is a prosthetic acetabular cup and the surface is configured to accommodate a prosthetic femoral head.

6. The orthopaedic implant of Claim 1 wherein the implant is a tibial bearing component and the surface is configured to accommodate sliding engagement with a prosthetic femoral knee component.

7. The implant of Claim 5 wherein the acetabular cup defines an inner diameter for accommodating a prosthetic femoral head and an outer periphery, wherein the outer periphery includes a porous layer.

8. The implant of Claim 7 wherein the porous layer is titanium which has been plasma flame sprayed to the outer periphery.

9. The implant of Claim 7 wherein the porous layer is fiber metal mesh.

10. An orthopaedic bearing component having a surface for articulating contact with a second component, wherein said surface is formed from a poly aryl ether ketone material.

11. A method of forming a prosthetic bearing surface, the method comprising the steps of,
a). providing a semi crystallin thermal plastic material capable of being injected in a fluid state into a mold;
b). providing a mold having a cavity the size and shape of the prosthetic bearing component;
c) injecting the semi crystallin thermal plastic material into the cavity in a fluid state at a predetermined pressure and temperature;
d). cooling the mold and semi crystallin thermal plastic material such that the material is in a solid state; and
e) removing the prosthetic bearing component from the mold.

12. The method of Claim 11 further including the step of forming a porous surface layer or a portion of the prosthetic bearing component by spraying a molten material on the prosthetic bearing component such that the molten material temporarily melts the portion of the prosthetic bearing component.

13. The method of Claim 11 wherein the semi crystallin thermal plastic material is a poly aryl ether ketone.

14. An orthopaedic implant formed entirely from a poly aryl ether ketone material.

15. The implant of Claim 14 further wherein said implant defines a bearing surface for articulating contact with a second component, said implant further including a porous metal surface layer attached to a surface of the implant spaced from the bearing surface.
